Europäisches Patentamt

European Patent Office

Office européen des brevets

(11) Numéro de publication : **0 481 903 A1**

## (12) DEMANDE DE BREVET EUROPEEN

(21) Numéro de dépôt : **91420369.0**

(51) Int. Cl.⁵ : **C12P 19/18**

(22) Date de dépôt : **18.10.91**

(30) Priorité : **18.10.90 FR 9013145**

(43) Date de publication de la demande :
**22.04.92 Bulletin 92/17**

(84) Etats contractants désignés :
**AT BE CH DE DK ES FR GB GR IT LI LU NL SE**

(71) Demandeur : **ORSAN**
**16 rue Ballu**
**F-75009 Paris (FR)**
(71) Demandeur : **MERCIAN CORPORATION**
**5-8, Kyobashi 1-chome**
**Chuo-ku, Tokyo (JP)**

(72) Inventeur : **Cami, Pierre**
**19 rue du Faubourg St Nicolas**
**F-80190 Nesle (FR)**
Inventeur : **Majou, Didier**
**7 rue Edouard Nortier**
**F-92200 Neuilly sur Seine (FR)**

(74) Mandataire : **Tilloy, Anne-Marie**
**IXAS Conseil, 15, rue Emile Zola**
**F-69002 Lyon (FR)**

(54) **Procédé de fabrication de cyclodextrines.**

(57) L'invention concerne un procédé de production de cyclodextrines caractérisé en ce qu'on fait réagir de l'amidon avec la cyclodextrine-glycosyl-transférase produite par Bacillus ohbensis (FERM BP-3180) ou produite par des souches obtenues à partir de Bacillus ohbensis. (FERM BP-3180) par mutation ou produite par d'autres bactéries dans lesquelles le gène de B. ohbensis codant pour la synthèse de CGTase a été introduit et en ce que les cyclodextrines sont extraites du milieu réactionnel par ultrafiltration.

EP 0 481 903 A1

Jouve, 18, rue Saint-Denis, 75001 PARIS

La présente invention concerne un procédé de production de cyclodextrines.

Plus particulièrement, l'invention concerne un procédé de production de cyclodextrines qui comprend la réaction de la cyclodextrine-glycosyl-transférase (CGTase) produite par Bacillus ohbensis (enregistré au FERMENTATION RESEARCH INSTITUTE au JAPON (FRI), antérieurement sous la référence FERM P 1990 et depuis 1981 sous la référence FERM BP-3180) ou produite par des souches obtenues à partir de Bacillus ohbensis (FERM BP-3180) par mutation ou produite par d'autres bactéries dans lesquelles le gène de B. ohbensis codant pour la synthèse de CGTase a été introduit, avec de l'amidon, pour décomposer l'amidon et recueillir ensuite les cyclodextrines formées (désignées ci-après par CD), à savoir, la bétacyclodextrine (désignée ci-après par béta-CD) et la gamma-cyclodextrine (désignée ci-après par gamma-CD).

La béta-CD est une molécule cyclique, composée de 7 unités glucopyranoses liées en 1-4 ; la gamma-CD est une molécule cyclique composée de 8 unités glucopyranoses liées en 1-4. Ces molécules présentent une cavité centrale hydrophobe permettant la réalisation de composés d'inclusion avec des molécules-hôtes. Les liaisons mises en jeu sont de faible énergie : le phénomène d'inclusion est donc réversible.

Les CD trouvent un champ d'application particulièrement intéressant notamment pour l'encapsulation d'arômes, de vitamines, de colorants, d'acides gras, de principes actifs pharmaceutiques, de pesticides et de fongicides.

Il est connu dans la technique de produire de la béta-CD et de la gamma-CD en utilisant la CGTase produite par Bacillus ohbensis.

Une nouvelle alpha-amylase, à savoir la CGTase produite par Bacillus ohbensis, a été pour la première fois décrite dans le brevet japonais 902415 (demande enregistrée sous le n°7335774). En outre, ce brevet indique la préparation de béta-CD et d'alpha-cyclodextrine (ci-après désignée alpha-CD)-(molécule cyclique composée de 6 unités glucopyranoses liées en 1-4) par réaction de l'amidon liquéfié ou hydrolysé avec la CGTase produite par Bacillus ohbensis.

L'article intitulé "Determination of CGTase from Bacillus ohbensis and its optimum pH using HPLC" paru dans le journal "Agricultural and Biological Chemistry (49(4), p. 1189 à 1191, 1985) enseigne que le pH optimal de la CGTase produite par Bacillus ohbensis se situe à pH 5,5. Egalement, cet article précise que l'analyse par chromatographie liquide à haute pression (HPLC) permet de mesurer directement la quantité de cyclodextrine formée et qu'en outre, cette méthode d'analyse est la plus fiable.

L'article intitulé "Comparative studies of CGTases from Bacillus ohbensis, Bacillus macerans Bacillus circulant and production of cyclodextrins using those CGTases" paru dans le journal "J.Jpn. Soc. Starch Sci." p. 144 à 151 (1986) enseigne que :
– le pH optimum de la CGTase produite par Bacillus ohbensis se situe à pH 5,5 ;
– la CGTase produite par Bacillus ohbensis, lorsqu'elle est amenée à réagir sur l'amidon liquide, produit de la béta-CD et de la gamma-CD ;
– à partir d'amidon liquéfié et de CGTase produite par Bascillus ohbensis, un rendement de 21,6 % en poids de CD est obtenu à pH variant entre 5 et 10.

La demande de brevet européen 220719 décrit un procédé de préparation de cyclodextrines (alpha, béta et gamma) selon lequel on fait réagir l'amidon liquéfié avec la CGTase produite par Bacillus ohbensis à un pH de préférence compris entre 6,5 et 7,5.

Les cyclodextrines formées sont extraites du milieu réactionnel par adsorption sur une résine insoluble dans l'eau et possédant un ligand de dimension telle, qu'il puisse se loger dans la cavité centrale des cyclodextrines et ainsi réaliser des composés d'inclusion.

Ce procédé permet de déplacer la réaction de formation des cyclodextrines tout en purifiant ces produits qui sont ensuite élués à l'eau chaude ou à l'aide d'un mélange d'eau et d'alcool : la concentration en cyclodextrines de ces solutions aqueuses n'excède pas 8 g/l.

Un but de l'invention est de proposer un procédé de préparation de béta-CD et de gamma-CD qui soit productif et relativement simple à mettre en oeuvre.

De façon surprenante, pour atteindre ce but, on a trouvé un procédé de production de cyclodextrines caractérisé en ce qu'on fait réagir de l'amidon, non dégradé, avec la cyclodextrine-glycosyl-transférase, produite par Bacillus ohbensis (FERM BP-3180) ou produite par des souches obtenues à partir de Bacillus ohbensis (FERM BP-3180) par mutation ou produite par d'autres bactéries dans lesquelles le gène de B. ohbensis codant pour la synthèse de CGTase a été introduit dans une solution aqueuse à pH compris de préférence entre 6,5 et 8,8 et en ce que les cyclodextrines produites sont extraites du milieu réactionnel par ultrafiltration.

Dans un souci de clarté, on désignera dans la suite de la description, par "enzyme B. O.", la cyclodextrine-glycosyl-transférase produite aussi bien par Bacillus ohbensis (FERM BP-3180) que par des souches obtenues à partir de Bacillus ohbensis (FERM BP-3180) par mutation ou produite par d'autres bactéries dans lesquelles le gène de B. ohbensis codant pour la synthèse de CGTase a été introduit.

A titre d'exemple de bactéries autres que B. ohbensis dans lesquelles le gène de B. ohbensis codant pour la synthèse de CGTase a été introduit, on peut citer la souche <u>Escherichia coli</u> décrite dans Appl. Microbiol Biotechnol (1991) 35 : p. 600 à 605.

Avantageusement, le procédé selon l'invention est mis en oeuvre de façon continue

Ce procédé présente l'avantage de permettre la production de béta-CD avec un rendement, par rapport à l'amidon mis en oeuvre, élevé et voisin de 40 %, voire supérieur.

L'une des caractéristiques principales de l'invention réside dans le fait que la réaction est effectuée à partir d'amidon "brut", c'est-à-dire de l'amidon qui n'est pas dégradé chimiquement, thermiquement ou à l'aide d'une enzyme telle qu'une amylase. En effet, contrairement aux procédés bien connus, grâce au procédé selon l'invention, l'amidon n'a pas besoin d'être traité (gélatinisé, liquéfié ou dextrinisé) préalablement à sa conversion enzymatique. L'amidon "brut" utilisé peut se présenter sous forme pulvérulente ou sous la forme d'un lait d'amidon, c'est-à-dire d'une suspension aqueuse.

Quand l'amidon est sous forme pulvérulente, on choisit de préférence de l'amidon de granulométrie n'excédant pas 200 μ. Sinon, on prévoit un système de protection du système d'ultrafiltration permettant d'éviter l'obstruction de ce dernier.

N'importe quel amidon peut être utilisé. A titre d'exemple, on peut citer l'amidon de pomme de terre, l'amidon de mais, l'amidon de blé et l'amidon de manioc. De préférence, on utilise la fécule de pomme de terre. Avantageusement, la concentration d'amidon dans le milieu réactionnel n'excède pas 15 % en poids, et de préférence encore, elle est comprise entre 8 % et 10 % en poids.

Conformément au procédé selon l'invention, le pH du milieu réactionnel est de préférence compris entre 6,5 et 8,8. En deçà, il se produit une diminution importante du débit d'ultrafiltration qui provient du colmatage de la membrane d'ultrafiltration Par voie de conséquence, la productivité chute également.

Le pH est avantageusement ajusté selon la provenance de l'amidon non dégradé. Les meilleurs rendements et productivités de béta-CD, à partir d'amidon de blé non dégradé, ont été atteints avec un pH compris entre 7 et 7,5.

A partir d'amidon de pomme de terre non dégradé, le pH du milieu réactionnel est avantageusement compris entre 7,5 et 8,8.

Les meilleurs rendements et productivités de béta-CD, à partir d'amidon de pomme de terre non dégradé, ont été atteints avec un pH compris entre 8 et 8,5.

Pour empêcher une inactivation prématurée de l'enzyme B. O., la température réactionnelle ne doit pas dépasser 65°C, et on la maintient de préférence entre 55°C et 60°C.

De préférence, l'activité de l'enzyme B.O. dans le temps est stabilisée à l'aide de sels de calcium tels que le chlorure de calcium. L'apport en calcium dans le milieu réactionnel peut être réalisé en utilisant de l'eau non déminéralisée ou en ajoutant des sels de calcium, à raison d'environ 0,01 % en poids.

Pour bénéficier d'une bonne activité enzymatique, et par conséquent assurer une bonne productivité en CD, la concentration en enzyme B. O. est de préférence comprise entre 1 et 10 unités par gramme d'amidon mis en oeuvre, mesurée par chromatographie HPLC, selon le mode opératoire décrit dans l'article "Determination of CGTase from <u>Bacillus ohbensis</u> and its optimum pH using HPLC" dans "Agricultural Biological Chemistry (49 (4), p. 1189 à 1991 - 1985-) : une unité d'enzyme B.O. est définie comme la quantité d'enzyme produisant 1 micromole de cyclodextrines en 1 minute à 50°C.

L'enzyme B.O. est plus spécifique de la production de béta-CD. Toutefois, l'amidon soumis à l'action de l'enzyme B. O. produit également la gamma-CD.

Le procédé selon l'invention peut être utilisé pour la production de gamma-CD. Par contre, la production d'alpha-CD, à partir d'amidon et d'enzyme B.O., est pratiquement nulle.

Au fur et à mesure de leur formation, les cyclodextrines (béta-CD et gamma-CD) sont extraites du milieu réactionnel par ultrafiltration, tandis que l'enzyme, l'amidon et l'amidon dextrinisé par l'enzyme B. O. sont retenus dans le milieu réactionnel.

La quantité d'eau dans le milieu réactionnel est maintenue quasiment constante dans le réacteur et on peut maintenir la concentration en amidon par addition successive ou addition en continu.

L'ultrafiltration est généralement réalisée à l'aide d'un dispositif d'ultrafiltration associé au réacteur dans lequel s'effectue la conversion enzymatique.

Le dispositif d'ultrafiltration comprend essentiellement une membrane, de préférence tubulaire, dont le point de coupure est supérieur à la masse moléculaire de la gamma-CD et inférieur à la masse moléculaire de l'enzyme B.O.

De préférence, on choisit une membrane dont le point de coupure est situé entre 2 000 et 30 000 Dalton. La membrane d'ultrafiltration convenant à l'invention peut être organique ou minérale. En outre, la membrane d'ultrafiltration peut être plane ou constituée de fibres creuses.

Parmi les membranes minérales convenant au procédé selon l'invention, on peut citer les membranes constituées d'un support en carbone et d'un filtre en zircone ou les membranes en céramiques.

Parmi les membranes organiques convenant à l'invention, on peut citer les membranes en polysulfone, en polyacrylonitrile, en polymères fluorés ou cellulosiques, par exemple les membranes planes de dénomination commerciale DDS dont le point de coupure est supérieur à 2 000 Dalton, ou les fibres creuses de dénomination commerciale ROMICON.

La productivité optimum du système constitué du réacteur et du système d'ultrafiltration est obtenue pour des débits d'ultrafiltration élevés et des concentrations en béta-CD dans le perméat maintenues à des valeurs comprises entre 10 g/l et 20 g/l. C'est en effet dans ces conditions que les meilleurs résultats sont obtenus. De préférence, on impose à la membrane d'ultrafiltration une pression transmembranaire comprise entre 1 et 2 bar. De préférence, on prévoit un réacteur dans lequel on peut ajuster le volume de réaction dans un rapport de l'ordre de 1 à 5 par rapport au volume total du réacteur, ce qui permet d'obtenir, suivant les conditions opératoires, le temps de séjour optimum. De préférence encore, on prévoit un système d'ultrafiltration tel que le temps de renouvellement du volume de réaction soit de 1/2 heure à 2 heures, la concentration moyenne en béta-CD dans le perméat étant maintenue à une valeur comprise entre 8 g/l et 15 g/l. Dans ces conditions, le procédé selon l'invention permet d'atteindre une productivité supérieure à 0,5 kg de béta-CD par heure et par m2 de membrane installée et pouvant atteindre 1 kg/h. m2.

Conformément à un second mode de réalisation de l'invention, le procédé selon l'invention est mis en oeuvre de façon discontinue de la manière suivante :

– on met en contact l'amidon en suspension agitée dans l'eau avec l'enzyme B. O. : la température et le pH sont régulés aux valeurs désirées et la concentration en amidon est ajustée à la valeur voulue. Cette première étape assure une parfaite maîtrise des paramètres du procédé et permet d'éviter, grâce au contrôle de l'addition d'amidon, la formation d'un gel qui, dans certains cas, peut conduire à la prise en masse dans le réacteur.

– Quand la viscosité souhaitée est atteinte, on met en service le système d'ultrafiltration et on régule la concentration dans le réacteur par addition d'eau et on extrait en continu les cyclodextrines produites dans le perméat. On n'ajoute plus d'amidon, ce qui conduit à réduire progressivement le volume de réaction

Le perméat obtenu est une solution aqueuse comprenant essentiellement de la béta-CD ainsi que de la gamma-CD et des dextrines de faible masse moléculaire.

Après élimination partielle d'eau du perméat, on obtient une solution aqueuse concentrée en béta-CD, gamma-CD et dextrines : cette solution peut être utilisée telle quelle pour préparer des complexes d'inclusion.

De préférence toutefois, on extrait la béta-CD du perméat. La récupération de béta-CD du perméat peut être effectuée par concentration du perméat suivie d'une cristallisation. La concentration du perméat peut être réalisée par évaporation sous pression réduite ou par osmose inverse. La cristallisation est réalisée par évaporation suivie d'un refroidissement.

Après séparation des cristaux de béta-CD, les gamma-CD peuvent être extraites des eaux-mères de cristallisation

D'autres caractéristiques et avantages de l'invention apparaîtront à la lecture des exemples qui suivent, donnés à titre illustratif et non limitatif de l'invention.

## EXEMPLE 1

Dans un réacteur, sous agitation, on met progressivement en suspension dans de l'eau brute, 3 680 g de fécule de pomme de terre correspondant à une concentration à 8 % en poids d'amidon. On régule la température à 58°C $^{+}$/- 2°C et le pH à 8,2 $^{+}$/- 0,2 et on introduit l'enzyme B. O. à raison de 2,5 unités par gramme d'amidon.

Dès la production de cyclodextrines dans le milieu réactionnel, on alimente à partir du réacteur un système d'ultrafiltration. La membrane d'ultrafiltration utilisée est une membrane minérale dont le support est à base de carbone et dont le filtre est en zircone. Elle possède un point de coupure de 15 000 Dalton et un débit à l'eau de 800 l/h. m² sous 4 bar à 25°C. On règle la pression de sorte à obtenir un débit moyen d'ultrafiltration de 65 l/h.m². Le débit d'ultrafiltration reste stable dans le temps.

Le rétentat d'ultrafiltration est recyclé dans le réacteur et on récupère les cyclodextrines dans le perméat.

Dans le réacteur, on maintient le niveau de la solution constituant le milieu réactionnel par addition d'eau et on maintient également la concentration par addition d'amidon.

Le perméat est concentré par évaporation puis on cristallise les cyclodextrines dans un cristalliseur. On obtient 1 100 g de béta-CD en cristaux blancs.

Le rendement de production de béta-CD sur l'amidon mis en oeuvre est égal à 29,9 % après 7 heures de réaction.

## EXEMPLE 2

Le mode opératoire suivi dans cet exemple est le même que celui de l'Exemple 1 avec les différences suivantes :

- amidon de pomme de terre non dégradé : 262 kg  (soit 10 % en poids dans le
  mis en oeuvre                                              milieu réactionnel)
- enzyme B.O.                          : 3 unités/gramme
- débit moyen d'ultrafiltration        : 56,4 l/h.m$^2$ de membrane

Les résultats obtenus sont les suivants :
– 104 kg de béta-CD après 40 heures de réaction
– rendement en béta-CD égal à 39,7 % par rapport à l'amidon mis en oeuvre.
Simultanément à la production de béta-CD, il y a production de gamma-CD. La solubilité de la béta-CD dans l'eau étant différente de celle de la gamma-CD, on sépare la béta-CD de la gamma-CD par concentration et cristallisation du perméat.
– On obtient 25 kg de gamma-CD dans les eaux-mères de cristallisation.
– Le rendement en gamma-CD par rapport à l'amidon mis en oeuvre est égal à 9,5 %.

## EXEMPLE 3

Le mode opératoire suivi dans cet exemple est le même que celui de l'Exemple 1 avec les différences suivantes :

- amidon de pomme de terre non dégradé : 350 kg  (soit 10 % en poids dans le
  mis en oeuvre                                              milieu réactionnel)
- enzyme B.O.                          : 3 unités/gramme
- débit moyen d'ultrafiltration        : 61,1 l/h.m$^2$ de membrane

Les résultats obtenus sont les suivants:
– 132 kg de béta-CD après 48 heures de réaction
– le rendement en béta-CD par rapport à l'amidon mis en oeuvre est égal à 37,7 %.
Simultanément à la production de béta-CD, il y a production de gamma-CD. La solubilité de la béta-CD dans l'eau étant différente de celle de la gamma-CD, on sépare la béta-CD de la gamma-CD par concentration et cristallisation du perméat.
– On obtient 31,7 kg de gamma-CD dans les eaux-mères de cristallisation.
– Le rendement en gamma-CD par rapport à l'amidon mis en oeuvre est égal à 9,0 %.

## EXEMPLE 4

Le mode opératoire suivi dans cet exemple est le même que celui de l'Exemple 1 avec les différences suivantes :

- amidon de pomme de terre non dégradé : 1 322 g
  mis en oeuvre (poids sec)

- enzyme B.O.                          : 3 unités/gramme
- pH                                   : 8,2
- température                          : 58°C
- débit moyen d'ultrafiltration        : 15 l/h.m$^2$ de membrane

Egalement diffère la nature de la membrane qui est, dans cet exemple, une membrane organique DDS à point de coupure égal à 6 000 Dalton.

Les résultats de cet exemple sont :

– béta-CD : 476 g

– le rendement en béta-CD par rapport à l'amidon mis en oeuvre est égal à 36 % après 6 heures 30 de réaction.

## EXEMPLE 5

Le mode opératoire suivi dans cet exemple est le même que celui de l'Exemple 1 avec les différences suivantes :

- amidon de pomme de terre non dégradé mis en oeuvre (poids sec) : 3 555 g
- enzyme B.O. : 3 unités/gramme d'amidon
- pH : 8,6
- température : 58°C
- débit moyen d'ultrafiltration : 26 l/h.m$^2$ de membrane

Egalement diffère la nature de la membrane qui est dans cet exemple une membrane de dénomination commerciale SCT en céramique dont le diamètre moyen des pores est de 200 Ängströms.

Les résultats de cet exemple sont :

– 824 g de béta-CD produite après 5 h de réaction.

– le rendement en béta-CD par rapport à l'amidon consommé est égal à 42 % après 5 h de réaction.

## EXEMPLE 6

Le mode opératoire suivi dans cet exemple est le même que celui de l'Exemple 1 avec les différences suivantes :

- amidon de pomme de terre non dégradé mis en oeuvre : 435 kg (soit 10 % en poids dans le milieu réactionnel)
- enzyme B.O. : 3 unités/gramme
- pH : 7,2
- débit moyen d'ultrafiltration : 59 l/h.m$^2$ de membrane

Les résultats obtenus sont les suivants:

– 35 kg de béta-CD après 38 heures de réaction

– le rendement en béta-CD par rapport à l'amidon mis en oeuvre est égal à 8 %.

## EXEMPLE 7

On utilise un réacteur agité associé à un système d'ultrafiltration 2S7 de la société Techsep, muni de membranes minérales dont le support est à base de carbone et dont le filtre est en zircone, de point de coupure 15 000 Dalton.

On met en oeuvre 1 800 grammes d'amidon de blé non-traité d'extrait sec 85 %, dans un volume total de 20 litres d'eau brute. On régule la température du milieu réactionnel a 60°C.

On réalise ainsi 4 essais en utilisant à chaque essai 4 630 unités d'enzyme B.O.

| Essais | a) | b) | c) | d) |
|---|---|---|---|---|
| Débit d'ultrafiltration  (l/h) | 30 | 20 | 20 | 20 |
| pH régulé à | 8,2 | 8 | 7,5 | 7,5 |
| Addition d'amidon (g) | 5 x 500 | 5 x 300 | 4 x 300 | 4 x 250 |
| Durée de l'essai (h) | 8 | 7 | 7h 30 | 7h 30 |

Les additions d'amidon sont réalisées chaque heure, la première étant faite une heure après le début de production. Le perméat est recueilli en continu, le niveau de liquide dans le réacteur étant maintenu constant par addition d'eau brute.

| Résultats obtenus | a) | b) | c) | d) |
|---|---|---|---|---|
| Béta-CD produites (g) | 1 011 | 986 | 882 | 780 |
| Gamma-CD produites (g) | 221 | 218 | 253 | 217 |
| Rendement Béta-CD/ amidon consommé | 31 | 36 | 41 | 38,4 |

Les valeurs de la productivité et du rendement de la réaction réalisée à partir d'amidon de blé sont similaires à celles réalisées à partir d'amidon de pomme de terre.

**EXEMPLE 8**

On utilise le même appareillage qu'à l'exemple 7, mais on utilise cette fois de l'amidon de maïs ; 1 800 g pour 20 litres d'eau, température 60°C, pH régulé à 8, mise en oeuvre de 3 850 unités d'enzyme B.O.

On régule le débit de perméat à 20 l/h. En 15 heures, on ajoute 5 000 g d'amidon de maïs.

On produit en 15 heures, 610 g de béta-CD et 180 g de gamma-CD. Le rendement en béta-CD sur amidon sec consommé est mesuré égal à 18 %.

**Revendications**

**1 -** Procédé de production de béta-cyclodextrine et de gamma-cyclodextrine caractérisé en ce qu'on fait réagir de l'amidon " brut", c'est-à-dire de l'amidon non dégradé, avec la cyclodextrine-glycosyl-transferase, produite par <u>Bacillus ohbensis</u> (FERM BP-3180) ou produite par des souches obtenues à partir de <u>Bacillus ohbensis</u> (FERM BP-3180) par mutation ou produite par d'autres bactéries dans lesquelles le gène de <u>B. ohbensis</u> codant pour la synthèse de CGTase a été introduit et en ce que les cyclodextrines sont extraites du milieu réactionnel par ultrafiltration.

**2 -** Procédé selon la revendication 1, caractérisé en ce que le pH du milieu réactionnel est compris entre 6,5 et 8,8.

**3 -** Procédé selon la revendication 1 ou 2, caractérisé en ce qu'on utilise comme amidon, l'amidon de pomme de terre, l'amidon de maïs, l'amidon de blé ou l'amidon de manioc.

**4 -** Procédé selon la revendication 1, 2 ou 3, caractérisé en ce qu'on utilise une concentration d'amidon n'excédant pas 15 % en poids, et de préférence comprise entre 8 % et 10 % en poids.

**5 -** Procédé selon la revendication 1, 2, 3 ou 4, caractérisé en ce que la réaction est effectuée à partir d'amidon de blé non dégradé, à pH compris entre 7 et 7,5.

**6 -** Procédé selon la revendication 1, 2, 3 ou 4, caractérisé en ce que la réaction est effectuée à partir d'amidon de pomme de terre non dégradé, à pH compris entre 7,5 et 8,8.

**7 -** Procédé selon la revendication 6, caractérisé en ce que la réaction est effectuée à partir d'amidon de pomme de terre non dégradé, à pH compris entre 8 et 8,5.

**8 -** Procédé selon l'une quelconque des revendications 1 à 7, caractérisé en ce que la réaction est effectuée à une température n'excédant pas 65°C, et de préférence comprise entre 55°C et 60°C.

**9 -** Procédé selon l'une quelconque des revendications 1 à 8, caractérisé en ce que la réaction est effectuée avec une concentration en cyclodextrine-glycosyl-transferase comprise entre 1 et 10 unités par gramme d'amidon mis en oeuvre.

**10 -** Procédé selon l'une quelconque des revendications 1 à 9, caractérisé en ce que le système constitué par le réacteur et le système d'ultrafiltration permet d'ajuster le temps de séjour moyen de l'amidon mis en oeuvre et permet d'ajuster le temps de renouvellement du volume de réaction à une valeur comprise entre 1/2 heure et 2 heures, la concentration moyenne en béta-CD dans le perméat étant maintenue à une valeur comprise entre 8 g/l et 15 g/l.

EP 0 481 903 A1

**Office européen des brevets**

**RAPPORT DE RECHERCHE EUROPEENNE**

Numero de la demande

EP 91 42 0369

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | AGRIC. BIOL. CHEM., vol. 49, no. 4, 1985, pages 1189-1191, Tokyo, JP; M. SATO et al.: "Determination of CGTase from Bacillus ohbensis and its optimum pH using HPLC" * Figures 1,3,5 * | 2,4-9 | C 12 P 19/18 |
| A | RESEARCH AND DEVELOPMENT IN JAPAN AWARDED THE OKOCHI MEMORIAL PRIZE, 1982, pages 47-52, Okochi Memorial Foundation, JP; K. HORIKOSHI et al.: "Alkalophilic microorganisms and new fermentation techniques - industrial production of cyclodextrins" * Figure 2; page 50, lignes 4-8; page 51, alinéa 2 * | 1-8,10 | |
| A | US-A-4 477 568 (H. HOKSE et al.) * Résumé; figure * | 1 | |
| D,A | EP-A-0 220 719 (SANRAKU) * Example 13 * | 1 | **DOMAINES TECHNIQUES RECHERCHES (Int. Cl.5)** C 12 P C 12 N |

Le présent rapport a été établi pour toutes les revendications

| Lieu de la recherche | Date d'achèvement de la recherche | Examinateur |
|---|---|---|
| LA HAYE | 29-11-1991 | VAN DER SCHAAL C.A.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

9